# FASCICULE DE BREVET EUROPEEN

(11) **EP 1 366 816 B1**
(45) Date de publication et mention de la délivrance du brevet: **05.08.2009**
(21) Numéro de dépôt: 03291105.9
(22) Date de dépôt: 09.05.2003
(51) Int. Cl.: B01J 31/18, C07F 15/04, C08F 10/00, C07C 2/32, C07D 213/30, C07D 233/64, C07D 263/14

(54) **Complexes organométalliques comportant des ligands chelatants bidentes associant un hétérocycle azoté avec un alcool et leur utilisation pour catalyser l'oligomérisation des oléfines**
Organometallkomplexe mit aus N-Heterocyclus und Alkohol bestehenden Bidentatchelatliganden, und deren Verwendung zur Olefinoligomerisation
Organometallic complexes containing N-hetereocycle and alcohol bidentate chelating ligands, and their use for olefin oligomerisation

(30) Priorité: 28.05.2002 FR 0206476
(43) Date de publication de la demande: 03.12.2003
(73) Titulaire: Institut Français du Pétrole, 92852 Rueil-Malmaison Cedex (FR)
(72) Inventeur: Speiser, Fredy, 22523 Hambourg (DE); Braunstein, Pierre, 67000 Strasbourg (FR); Saussine, Lucien, 78290 Croissy sur Seine (FR)

(56) Documents cités:
- US-A- 4 000 211
- US-A- 4 069 273
- C. BOLM ET AL.: "Catalytic enantioselective conjugate addition of dialkylzinc compounds to chalcones" CHEMISCHE BERICHTE., vol. 125, 1992, pages 1205-1215, XP002227949 VERLAG CHEMIE GMBH. WEINHEIM., DE ISSN: 0009-2940
- DESJARDINS S Y ET AL: "Insertion into the nickel-carbon bond of N-O chelated arylnickel(II) complexes. The development of single component catalysts for the oligomerisation of ethylene" JOURNAL OF ORGANOMETALLIC CHEMISTRY, ELSEVIER-SEQUOIA S.A. LAUSANNE, CH, vol. 515, no. 1, 31 mai 1996 (1996-05-31), pages 233-243, XP004036010 ISSN: 0022-328X
- DATABASE CA PLUS [en ligne] CHEMICAL ABSTRACTS SERVICE, COLUMBUS, OHIO, US; GOLOUNIN A.V. ET AL.: "Extractive power of mixtures of carboxylic acids and organic bases" retrieved from STN Database accession no. 1992:461739 XP002227950 & SIBIRSKII KHIMICHESKII ZHURNAL, vol. 4, 1991, pages 65-68,

## Description

La présente invention concerne l'oligomérisation des oléfines, en particulier de l'éthylène.

Un objet de l'invention est de fournir une nouvelle composition catalytique. Un autre objet de l'invention est de fournir un procédé d'oligomérisation des oléfines, en particulier de l'éthylène utilisant cette composition catalytique.

Il est bien connu que les monooléfines-α telles que l'éthylène, le propylène ou le butène-1 peuvent être oligomérisées avec des systèmes catalytiques à base de métaux de transition tels que le nickel, le chrome, le titane, le zirconium ou d'autres métaux, en présence d'un co-catalyseur tel qu'un composé d'hydrocarbylaluminium, un halogénure d'hydrocarbylaluminium ou un aluminoxane.

Il a été décrit plusieurs type de ligands pour stabiliser l'espèce catalytique et orienter la sélectivité de la réaction d'oligomérisation. Les ligands de type phosphine (US-A-4 518 814 et US-A-5 245 097) ou les ligands bidentés associant une phosphine avec un anion oxygéné tel qu'un carboxylate (US-A-3 676 523, US-A-4 472 522 , US-A-4 528 416, US-A-5 557 027), un énolate (W. Keim et al. Angew. Chem. Int. Ed. 1978, 17, 466, US-A-4 293 727 et US-A-4 310 716). Parmi ces catalyseurs, certains sont mis en oeuvre dans un alcool. Pour préparer le catalyseur, ll est possible de partir de nickel zéro valent (par exemple le nickel bis (cyclooctadiène)) ou de réduire le sel de nickel(ll) par un composé alkylaluminium ou un hydrure de bore.

Plus récemment, l'utilisation de complexes comportants des ligands chélatants monoanioniques de type salicylaldimine a été décrite par plusieurs groupes (Grubbs et al. in Organometallics, 1998, 17, 3149-51 ; WO-A-98/30609) uniquement pour la polymérisation de l'éthylène. Ces complexes sont préparés par réaction d'un composé organométallique du nickel avec les ligands chélatants ou les anions qui en dérivent. Des complexes du nickel préparés par réaction de l'anion picolinate sur le complexe Ni(o-tolyl)Br(PPh₃)₂ catalysent l'oligomérisation de l'éthylène avec une activité faible (oléfines C4-C10 : 75 % - sélectivité alpha : 63 % - K. S. Cavell et al. in J. Organomet. Chem. 1996, 515, 233-43).

US-A-4,069,273 décrit un catalyseur de dimérisation des oléfinies comprenant un complexe de nickel comprenant 2-hydroxymethyl pyridine comme ligand.

Il a maintenant été trouvé, de façon inattendue, qu'une composition catalytique comprenant au moins un complexe du nickel contenant au moins un ligand chélatant bidenté associant un hétérocycle azoté avec un alcool et au moins un composé d'hydrocarbylaluminium ou au moins un aluminoxane, nommé ici agent activateur, présente une activité améliorée pour l'oligomérisation des oléfines, en particulier de l'éthylène.

Ainsi, la composition catalytique de l'invention est définie comme comprenant :
- au moins un complexe de nickel contenant au moins un ligand chélatant bidenté associant un hétérocycle azoté avec un alcool ; et
- au moins un composé d'hydrocarbylaluminium choisi dans le groupe formé par les composés tris(hydrocarbyl)aluminium et les composés chlorés ou bromés d'hydrocarbylaluminium ou au moins un aluminoxane.

Plus précisément, les complexes de nickel mis en oeuvre dans cette invention ont pour formule générale L₂NiX₂ dans laquelle :
- L est un ligand chélatant bidenté associant un hétérocycle azoté avec un alcool et répondant à la formule générale :

   Ha∼C(OH)RR'

   dans laquelle R et R', identiques ou différents, représentent chacun un radical hydrocarboné monovalent contenant jusqu'à 12 atomes de carbone, tel que alkyle, aryle, aralkyle, alkaryle, cycloalkyle ou aryle substitué, ou un radical perfluoroalkyle et l'hétérocycle azoté Ha étant choisi parmi les dérivés comportants les cycles pyridine, oxazoline ou imidazole, éventuellement substitués ;
- X peut être un radical alkyle, un anion halogénure, Cl, Br ou l, un anion acétylacétonate, acétate, trifluoroacétate ou plus généralement un anion carboxylate R"COO- pour lequel R" est un radical hydrocarbyle, par exemple alkyle, cycloalkyle, alkényle, aryle, aralkyle ou alkaryle contenant jusqu'à 20 atomes de carbone, de préférence un radical hydrocarbyle de 5 à 20 atomes de carbone, éventuellement substitué par des atomes d'halogène (fluor ou chlore). A titre d'exemples non limitatifs, l'anion carboxylate peut être choisi parmi les anions suivants : octoate, éthyl-2 hexanoate, stéarate, oléate, naphténate et adipate.

Plus précisément, les hétérocycles azotés Ha peuvent répondre aux formules générales ci-dessous : 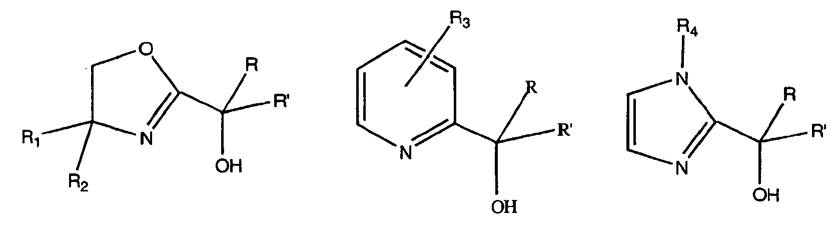 dans lesquelles les radicaux R₁, R₂, R₃ et R₄, qui peuvent être identiques ou différents, sont choisis parmi l'atome d'hydrogène, les radicaux alkyles linéaires ou ramifiés, les radicaux aryles, aralkyles ou alkaryles comportant de 1 à 12 atomes de carbone. Le radical R₃ peut être sur l'une quelconque des positions libres du noyau aromatique. A titre d'exemples non limitatifs, ces substituants peuvent être choisis parmi les radicaux méthyle, éthyle, isopropyle, isobutyle, tert-butyle, cyclohexyle, phényle et benzyle.

Par ailleurs, dans les formules ci-dessus, les radicaux R et R', R et R₃ ou R et R₄ peuvent être reliés entre eux et faire partie d'un même radical cyclique.

Pour les complexes L₂NiX_{2,} on peut citer à titre d'exemples non limitatifs les complexes suivants :

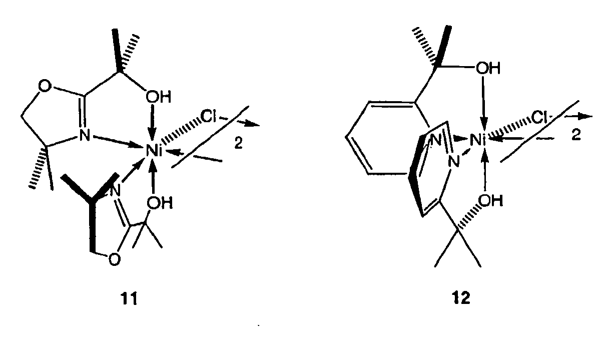

La préparation du complexe de nickel L₂NiX₂ se fait selon les méthodes connues dans la littérature pour la synthèse de complexes du nickel avec un ligand neutre. Tout procédé de préparation de ce composé peut convenir, comme par exemple la réaction du ligand avec un sel de nickel dans un solvant organique, par exemple un éther, un alcool, un solvant chloré, tel que le dichlorométhane. Le complexe peut être préparé *in situ* dans le solvant utilisé pour la réaction d'oligomérisation. Dans ce cas, l'ordre de mélange du sel de nickel et du ligand n'est pas critique. Cependant, on préfère préparer d'abord une solution d'un sel de nickel soluble en milieu organique, comme par exemple un carboxylate de nickel et ajouter ensuite le ligand.

Comme indiqué précédemment l'agent activateur mis en oeuvre peut être :
- soit un composé d'hydrocarbylaluminium choisi par exemple dans le groupe formé par les tris(hydrocarbyl)aluminium et les composés chlorés ou bromés d'hydrocarbylaluminium ;
- soit un aluminoxane.

Les composés tris(hydrocarbyl)aluminium et les composés chlorés ou bromés d'hydrocarbylaluminium répondent de préférence à la formule générale AIR"ₘY₃₋ₘ dans laquelle R" représente un radical hydrocarboné monovalent contenant par exemple jusqu'à 12 atomes de carbone tel que alkyle, aryle, aralkyle, alkaryle ou cycloalkyle, Y représente un atome d'halogène choisi par exemple parmi le chlore et le brome, Y étant de préférence un atome de chlore, m prend une valeur de 1 à 3, m étant de préférence égal à 1. Comme exemples de tels composés de formule AIR"ₘY₃₋ₘ, on peut mentionner le dichloroéthylaluminium, le sesquichlorure d'éthylaluminium, le chlorodiéthylaluminium, le triéthylaluminium, le dichloroisobutylaluminium, le chlorodiisobutylaluminium, le triisobutylaluminium et le tripropylaluminium.

Comme exemple d'aluminoxane, on peut mentionner le méthylaluminoxane.

Le complexe du nickel et le co-catalyseur d'aluminium optionnel peuvent être mis en contact dans un solvant constitué par un hydrocarbure saturé comme l'hexane, le cyclohexane, l'heptane, le butane ou l'isobutane, par un hydrocarbure insaturé comme une monooléfine ou une dioléfine comportant par exemple de 4 à 20 atomes de carbone, par un hydrocarbure aromatique tel que le benzène, le toluène, l'ortho-xylène, le mésitylène, l'éthylbenzène ou par un hydrocarbure chloré tel que le chlorobenzène ou le dichlorométhane, purs ou en mélange.

La concentration du nickel dans la solution catalytique varie en général de 1.10⁻⁵ à 0,1 mole/L, de préférence de 5.10⁻⁵ à 1.10⁻² mole/L.

Le rapport molaire entre l'hydrocarbylaluminium optionnel et le complexe de nickel est choisi entre 1/1 et 800/1, de préférence de 5/1 à 500/1.

Lorsque le composé d'hydrocarbylaluminium est utilisé, l'ordre de mélange des deux constituants de la composition catalytique n'est pas critique. Cependant, on préfère ajouter le composé d'hydrocarbylaluminium sur la solution du complexe.

L'invention comprend également un procédé d'oligomérisation des oléfines, en particulier de l'éthylène, mettant en oeuvre la composition catalytique précédente.

La réaction d'oligomérisation de l'éthylène peut être effectuée sous une pression totale de 0,5 à 15 MPa, de préférence de 1 à 8 MPa, et à une température de 20 à 180 °C, de préférence de 40 à 140 C.

Dans un mode particulier de mise en oeuvre de la réaction catalytique d'oligomérisation en discontinu, on introduit un volume choisi de la solution catalytique, constituée comme décrit ci-dessus, dans un réacteur muni des dispositifs habituels d'agitation, de chauffage et de refroidissement, puis on pressurise par de l'éthylène à la pression désirée, et on ajuste la température à la valeur souhaitée. Le réacteur d'oligomérisation est maintenu à pression constante par introduction d'éthylène jusqu'à ce que le volume total de liquide produit représente, par exemple, de 2 à 50 fois le volume de la solution catalytique primitivement introduit. On détruit alors le catalyseur par tout moyen habituel connu de l'homme du métier, puis on soutire et on sépare les produits de la réaction et le solvant.

En cas d'opération en continu, la mise en oeuvre est par exemple la suivante : la solution catalytique est injectée en même temps que l'éthylène dans un réacteur agité par les moyens mécaniques classiques ou par une recirculation extérieure, et maintenu à la température souhaitée. On peut aussi injecter séparément les composants du catalyseur dans le milieu réactionnel. L'éthylène est introduit par une vanne d'admission asservie à la pression, qui maintient celle-ci constante. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits de la réaction ainsi que le solvant sont séparés, par exemple par distillation. L'éthylène qui n'a pas été transformé peut être recyclé dans le réacteur.

Les exemples suivants illustrent l'invention sans en limiter la portée.

### EXEMPLE 1

### a) Préparation du complexe (oxazoline alcool)₂NiCl₂

Le ligand oxazoline alcool pour lequel R1 = R2 = R= R' = méthyle a été préparé selon les méthodes décrites dans la littérature (L.N Pridgen, G. M. Miller, J. Heterocyclic. Chem. 1983, 20, 1223).

Le ligand oxazoline alcool (0,700 g, 4,45 mmole) est mis en solution dans 30 mL de dichlorométhane, puis, après addition d'un demi équivalent de chlorure de nickel-diméthoxy-1,2-éthane, NiCl₂(DME) (0,474 g, 2,29 mmole), la solution est agitée pendant 12 heures. La solution est filtrée sur célite, puis concentrée. Le solide vert jaune est dissout dans 20 mL de toluène puis précipité par addition d'hexane, filtré, séché sous vide. Rendement : 0,911 g, soit 75 %. Ce produit est caractérisé par une bande d'absorption Infra-Rouge à 1662 cm⁻¹, caractéristique de la double liaison C=N coordinée au nickel.

### b) Oligomérisation de l'éthylène

Dans un ballon en verre de 100 mL placé sous atmosphère inerte, on introduit à l'abri de l'air et de l'humidité 0,063.10⁻³ mole de complexe de nickel, que l'on dilue avec 60 mL de toluène distillé et que l'on conserve sous atmosphère inerte.
Dans un autoclave en acier inoxydable d'un volume utile de 100 mL, muni d'une double enveloppe permettant de réguler la température par circulation d'huile, on introduit, dans l'ordre, sous atmosphère d'éthylène, et à la température ambiante, 10 mL de la solution du complexe de nickel préparée ci-dessus, soit 1,05.10⁻⁵ mole de nickel, et 2,1.10⁻³ mole de méthylaluminoxane en solution dans 10 mL de toluène. La température est alors portée à 30 °C et la pression d'éthylène est maintenue à 1 MPa.

Après 35 minutes de réaction, l'introduction d'éthylène est arrêtée et le réacteur est refroidi et dégazé, puis le gaz et le liquide qui a été soutiré au moyen d'une seringue sont analysés par chromatographie en phase vapeur. Les produits sont constitués en majorité d'oléfines alpha. La composition du mélange est donnée dans le Tableau 1.

### EXEMPLE 2

Dans le même appareillage que celui qui a été utilisé pour l'Exemple 1 et dans les mêmes conditions, à ceci près que le rapport méthylaluminoxane sur nickel est de 400/1 au lieu de 200/1. La composition des produits est donnée dans le Tableau 1.

### EXEMPLE 3

### a) Préparation du complexe de nickel

Pour la synthèse du complexe, on opère comme dans l'Exemple 1 à ceci près que l'on utilise le ligand pyridine alcool (avec R = R' = méthyle, R₃= H).

Synthèse du ligand pyridine alcool : il est préparé selon le mode opératoire décrit par Noyce et al. Dans J. Org. Chem 1973, 38, 2260.

### b) Oligomérisation de l'éthylène

On opère dans le même appareillage que celui qui a été utilisé pour l'Exemple 1 et dans les mêmes conditions. Le rapport méthylaluminoxane sur nickel est de 200/1. La composition des produits est donnée dans le Tableau 1.

### EXEMPLE 4

On opère comme dans l'Exemple 3, à ceci près que le rapport aluminoxane sur nickel est de 400/1 au lieu de 200/1.

### EXEMPLE 5

On opère dans le même appareillage que celui qui a été utilisé pour l'Exemple 1 et dans les mêmes conditions à ceci près que l'agent activateur est le dichloroéthylaluminium avec un rapport molaire dichloroéthylaluminium sur nickel de 6/1.

### EXEMPLE 6

On opère dans le même appareillage que celui qui a été utilisé pour l'Exemple 1 et dans les mêmes conditions à ceci près que l'on utilise le complexe de l'Exemple 3 et que l'agent activateur est le dichloroéthylaluminium avec un rapport molaire dichloroéthylaluminium sur nickel de 6/1.

**Tableau 1**

| Exemple | Répartition des oligomères (% poids) | | | Productivité (g d'éthylène/g de Ni /h) |
|---|---|---|---|---|
| | C4 | C6 | C8 | |
| 1 | 87,5 | 10,5 | 2,0 | 7 410 |
| 2 | 84,0 | 13,7 | 2,3 | 8 920 |
| 3 | 87,0 | 11,5 | 1,5 | 5 000 |
| 4 | 82,6 | 14,8 | 2,5 | 6 800 |
| 5 | 70,4 | 27,6 | 1,9 | 83 300 |
| 6 | 64,2 | 32,6 | 3,1 | 46 400 |

## Revendications

1. Composition catalytique **caractérisée en ce qu'**elle comprend au moins un complexe de nickel contenant au moins un ligand chélatant bidenté associant un hétérocycle azoté avec un alcool et au moins un composé d'hydrocarbylaluminium choisi parmi les composés tris(hydrocarbyl)aluminium et les composés chlorés ou bromés d'hydrocarbylaluminium ou au moins un aluminoxane.

2. Composition selon la revendication 1 **caractérisée en ce que** ledit complexe de nickel répond à la formule générale L₂NiX₂ dans laquelle :
- L est un ligand chélatant bidenté associant un hétérocycle azoté avec un alcool, de formule générale :
Ha∼C(OH)RR'
dans laquelle :
- R et R', identiques ou différents, représentent chacun un radical hydrocarboné monovalent contenant jusqu'à 12 atomes de carbone choisi parmi les radicaux alkyles, aryles, aralkyles, alkaryles, cycloalkyles ou aryles substitués, ou un radical perfluoroalkyle ;
- et les hétérocycles azotés Ha sont choisis parmi les dérivés comportant les cycles pyridine, oxazoline ou imidazole, ou un de ces hétérocycles substitués ;
- et X est un radical alkyle, un anion halogénure, acétylacétonate ou carboxylate.

3. Composition selon la revendication 2 **caractérisée en ce que,** dans le ligand chélatant bidenté, l'hétérocycle Ha est un cycle pyridine, oxazoline ou N-méthylimidazole ou un de ces hétérocycles substitués.

4. Composition selon la revendication 3 **caractérisée en ce que** le ligand chélatant bidenté répond à l'une des formules : 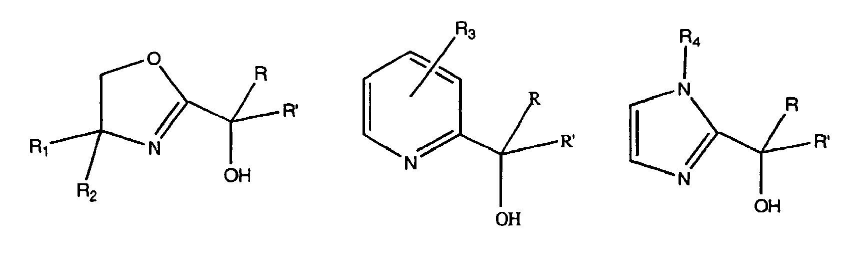 dans lesquelles les radicaux R₁, R₂, R₃ et R₄, identiques ou différents, sont choisis parmi l'atome d'hydrogène, les radicaux alkyles linéaires ou ramifiés, les radicaux aryles, aralkyles ou alkaryles comportant de 1 à 12 atomes de carbone, le radical R₃ étant placé sur l'une quelconque des positions libres du noyau aromatique.

5. Composition selon l'une des revendications 2 à 4 **caractérisée en ce que,** dans le complexe de nickel, X représente un anion Cl, Br, I, acétylacétonate, acétate ou trifluoroacétate.

6. Composition selon l'une des revendications 1 à 5 **caractérisée en ce que** le composé d'hydrocarbylaluminium est choisi parmi le dichloroéthylaluminium, le sesquichlorure d'éthylaluminium, le chlorodiéthylaluminium, le triéthylaluminium, le dichloroisobutylaluminium, le chlorodiisobutylaluminium, le triisobutylaluminium, le tripropylaluminium et le méthylaluminoxane.

7. Composition selon la revendication 6 **caractérisée en ce que** le composé d'hydrocarbylaluminium est le méthylaluminoxane.

8. Composition selon l'une des revendications 1 à 7 **caractérisée en ce que** les composants du catalyseur sont mis en contact dans un solvant constitué par un hydrocarbure saturé, insaturé oléfinique ou aromatique.

9. Composition selon l'une des revendications 1 à 8 **caractérisée en ce que** la concentration du nickel dans la solution catalytique est de 1.10⁻⁵ à 0,1 mole/L.

10. Composition selon l'une des revendications 1 à 9 **caractérisée en ce que** le rapport molaire entre l'hydrocarbylaluminium et le complexe du nickel est de 5/1 à 500/1.

11. Procédé d'oligomérisation de l'éthylène utilisant une composition catalytique selon l'une des revendications 1 à 10.

12. Procédé selon la revendication 11 **caractérisé en ce que** la réaction d'oligomérisation de l'éthylène est effectuée sous une pression de 0,5 à 15 MPa et à une température de 20 à 180 °C.

## Claims

1. A catalyst composition **characterized in that** it comprises at least one nickel complex containing at least a bidentate chelating ligand associating a nitrogen-containing heterocyclic with an alcohol and at least one hydrocarbylaluminium compound choosen among tris(hydrocarbyl)aluminum compounds and chlorinated or brominated hydrocarbylaluminum compounds or at least one aluminoxane.

2. A composition according to claim 1 **characterized in that** said nickel complex corresponds to general formula L₂NiX₂ in which :
- L is a bidentate chelating ligand associating a nitrogen-containing heterocyclic with an alcohol, of general formula:
Ha∼C(OH)RR'
in which:
- R and R', identical or different, each represent a monovalent radical that contains up to 12 carbon atoms wherein such monovalent radical is alkyl, aryl, aralkyl, alkaryl, cycloalkyl or a substituted aryl radical, or a perfluoroalkyl radical,
- and nitrogen-containing heterocyclic Ha are choosen among derivates that contains pyridine, oxazoline or an imidazole cycles; or one of these heterocycles that is substituted,
- and X is an alkyl radical, a halide anion, acetylacetonate or a carboxylate.

3. A catalyst composition according to claim 2, **characterized in that,** in the bidentate chelating ligand, nitrogen-containing heterocyclic Ha is a pyridine, oxazoline or N-methylimidazole cycle, or one of these substituted heterocycles that is substituted.

4. A catalyst composition according to claim 3, **characterized in that** the bidentate chelating ligand corresponds to one of the formulas : 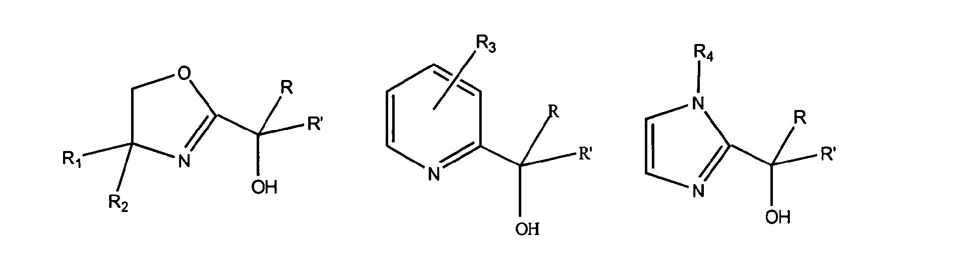 in which radicals R 1, R 2, R 3 and R 4, identical or different, are a hydrogen atom, a member having 1 12 carbon atoms being a linear or branched alkyl radical, an aryl, an aralkyl or an alkaryl radical having from 1 to 12 carbon atoms, the R3 radical being placed in any of the free positions of the aromatic core.

5. A catalyst composition according to any one of claims 2 to 4, **characterized**
**in that,** in the nickel complex, X is a Cl, Br, I, acetylacetonate, acetate or trifluoroacetate anion.

6. A catalyst composition according to any one of claims 1 to 5, **characterized**
**in that** the hydrocarbylaluminum compound is at least one of dichloroethyl aluminum, ethyl aluminum sesquichloride, chlorodiethyl aluminum, triethyl aluminum, dichloroisobutyl aluminum, chlorodiisobutyl aluminum, triisobutyl aluminum, tripropyl aluminum, or methylaluminoxane.

7. A catalyst composition according to claim 6 **characterized in that** the hydrocarbylaluminum compound is the methylaluminoxane.

8. A catalyst composition according to any one of claims 1 to 7, **characterized**
**in that** the components of the catalyst are brought into contact in a solvent comprising an olefinic or aromatic unsaturated or saturated hydrocarbon.

9. A catalyst composition according to any one of claims 1 to 8, **characterized**
**in that** the concentration of nickel in the catalytic solution is 1.10 ⁻⁵ to 0.1 mol/l.

10. A catalyst composition according to any one of claims 1 to 9, **characterized**
**in that** the molar ratio between hydrocarbylaluminum compound and the nickel complex is 5/1 to 500/1.

11. A process of oligomerization of ethylene using a catalyst composition according to to any one of claims 1 to 10.

12. A process according to claim 11, wherein the oligomerization reaction of the ethylene is carried out under a pressure of 0.5 to 15 MPa and at a temperature of 20 to 180° C.

## Patentansprüche

1. Katalytische Zusammensetzung, **dadurch gekennzeichnet, dass** sie mindestens einen Nickelkomplex, der mindestens einen chelatbildenden, zweizähnigen Liganden enthält, der einen stickstoffhaltigen Heterocyclus mit einem Alkohol assoziiert, und mindestens eine Hydrocarbylaluminiumverbindung, die aus Tris(hydrocarbyl)aluminiumverbindungen und chlorhaltigen oder bromhaltigen Hydrocarbylaluminiumverbindungen ausgewählt ist, oder mindestens ein Aluminoxan umfasst.

2. Zusammensetzung nach Anspruch 1, **dadurch gekennzeichnet, dass** der Nickelkomplex der allgemeinen Formel L₂NiX₂ genügt, worin:
- L ein chelatbildender, zweizähniger Ligand, der einen stickstoffhaltigen Heterozyklus mit einem Alkohol assoziiert, der allgemeinen Formel:
Ha∼C(OH)RR'
ist, worin:
- R und R' gleich oder unterschiedlich sind und jeweils für einen einwertigen Kohlenwasserstoffrest mit bis zu 12 Kohlenstoffatomen, der aus Alkyl-, Aryl-, Aralkyl-, Alkaryl-, Cycloalkyl- oder substituierten Arylresten ausgewählt ist, oder einen Perfluoralkylrest stehen;
- und die stickstoffhaltigen Heterocyclen Ha aus Derivaten ausgewählt sind, die Pyridin-, Oxazolin- oder Imidazolcyclen oder einen dieser Heterocyclen als substituierten Heterocyclus enthalten;
- und X ein Alkylrest, ein Halogenid-, Acetylacetonat- oder Carboxylat-Anion ist.

3. Zusammensetzung nach Anspruch 2, **dadurch gekennzeichnet, dass** im chelatbildenden, zweizähnigen Liganden der Heterozyklus Ha ein Pyridin-, Oxazolin- oder N-Methylimidazolcyclus oder einer dieser Heterocyclen als substituierter Heterocyclus ist.

4. Zusammensetzung nach Anspruch 3, **dadurch gekennzeichnet, dass** der chelatbildende, zweizähnige Ligand einer der folgenden Formeln genügt: 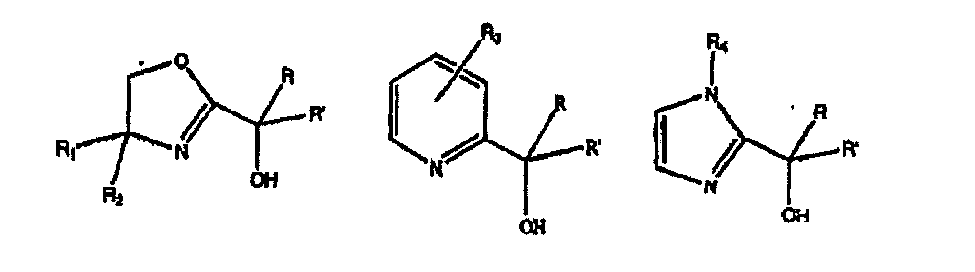 worin die Reste R₁, R₂, R₃ und R₄ gleich oder unterschiedlich sind und aus einem Wasserstoffatom, aus geradkettigen oder verzweigten Alkylresten, aus Aryl-, Aralkyl- oder Alkarylresten mit 1 bis 12 Kohlenstoffatomen ausgewählt sind, wobei der Rest R₃ an einer beliebigen der freien Stellen des aromatischen Kerns angeordnet ist.

5. Zusammensetzung nach einem der Ansprüche 2 bis 4, **dadurch gekennzeichnet, dass** im Nickelkomplex X für ein Cl-, Br-, I-, Acetylacetonat-, Acetat- oder Trifluoracetat-Anion steht.

6. Zusammensetzung nach einem der Ansprüche 1 bis 5, **dadurch gekennzeichnet, dass** die Hydrocarbylaluminiumverbindung aus Dichlorethylaluminium, Ethylaluminiumsesquichlorid, Chlordiethylaluminium, Triethylaluminium, Dichlorisobutylaluminium, Chlordiisobutylaluminium, Triisobutylaluminium, Tripropylaluminium und Methylaluminoxan ausgewählt ist.

7. Zusammensetzung Anspruch 6, **dadurch gekennzeichnet, dass** die Hydrocarbylaluminiumverbindung Methylaluminoxan ist.

8. Zusammensetzung nach einem der Ansprüche 1 bis 7, **dadurch gekennzeichnet, dass** die Komponenten des Katalysators in einem aus einem gesättigten, ungesättigten olefinischen oder aromatischen Kohlenwasserstoff gebildeten Lösungsmittel in Kontakt gebracht werden.

9. Zusammensetzung nach einem der Ansprüche 1 bis 8, **dadurch gekennzeichnet, dass** die Nickelkonzentration in der katalytischen Lösung 1.10⁻⁵ bis 0,1 mol/l beträgt.

10. Zusammensetzung nach einem der Ansprüche 1 bis 9, **dadurch gekennzeichnet, dass** das Molverhältnis zwischen dem Hydrocarbylaluminium und dem Nickelkomplex 5/1 bis 500/1 beträgt.

11. Verfahren zur Oligomerisation von Ethylen unter Verwendung einer katalytischen Zusammensetzung nach einem der Ansprüche 1 bis 10.

12. Verfahren nach Anspruch 11, **dadurch gekennzeichnet, dass** die Reaktion zur Oligomerisation von Ethylen bei einem Druck von 0,5 bis 15 MPa und einer Temperatur von 20 bis 180 °C durchgeführt wird.
